# EUROPEAN PATENT SPECIFICATION

(11) **EP 2 183 015 B1**
(45) Date of publication and mention of the grant of the patent: **25.01.2012**
(21) Application number: 08796971.3
(22) Date of filing: 31.07.2008
(51) Int. Cl.: A61M 25/09, A61M 25/00

(54) **Guidewires and methods for manufacturing guidewires**
Führungsdrähte und Verfahren zur Herstellung von Führungsdrähten
Fils guides et procédés pour fabriquer des fils guides

(30) Priority: 03.08.2007 US 833658
(43) Date of publication of application: 12.05.2010
(73) Proprietor: Boston Scientific Limited, Christ Church (BB)
(72) Inventor: SKUJINS, Peter, Minneapolis, Minnesota 55403 (US); JOHNSON, Dave B., Hopkins, Minnesota 55343 (US); VOELLER, Virgil F., St. Louis Park, Minnesota 55416 (US)
(74) Representative: Vossius & Partner
(86) International application number: PCT/US2008/071798
(87) International publication number: WO 2009/020836

(56) References cited:
- EP-A- 0 879 616
- EP-A- 1 698 370
- WO-A-96/38193
- WO-A-2008/034010
- US-A1- 2004 167 437

## Description

The present invention pertains to intracorporal medical devices, for example, intravascular guidewires, catheters, stents, and the like as well as improved methods for manufacturing medical devices. More particularly, the invention relates to guidewires and methods for making guidewires.

A wide variety of intracorporeal medical devices have been developed for medical use, for example, intravascular use. Some of these devices include guidewires, catheters, stents, and the like. For example, EP 1 698 370 discloses a guide wire comprising a core member having at least one projection projecting concentrically relative to an axis, and a coil having a distal end portion and a proximal end portion and covering at least a distal side portion of said core member, wherein said coil is fixed to said projection. Of the known medical devices, each has certain advantages and disadvantages. There is an ongoing need to provide alternative medical devices as well as alternative methods for manufacturing medical devices.

The invention provides design, material, and manufacturing method alternatives for medical devices.

The invention is directed to a device according to claim 1 and a method according to claim 12.

The above summary of some embodiments is not intended to describe each disclosed embodiment or every implementation of the present invention. The Figures, and Detailed Description, which follow, more particularly exemplify these embodiments.

The invention may be more completely understood in consideration of the following detailed description of various embodiments of the invention in connection with the accompanying drawings, in which:
Figure 1 is a plan view of an example medical device disposed in a blood vessel;
Figure 2 is a cross-sectional side view of a portion of an example medical device;
Figure 2A is a cross-sectional side view of a portion of another example medical device;
Figure 3 is a cross-sectional side view of a portion of another example medical device;
Figure 4 is a cross-sectional side view of a portion of another example medical device;
Figure 4A is a cross-sectional side view of a portion of another example medical device;
Figure 5 is a cross-sectional side view of a portion of another example medical device;
Figure 6 is a cross-sectional side view of a portion of another example medical device; and
Figure 7 is a cross-sectional side view of a portion of another example medical device.

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

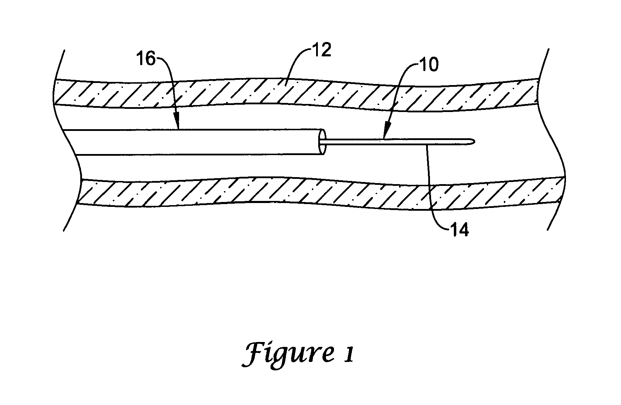

Figure 1 is a plan view of an example guidewire 10 disposed in a blood vessel 12. Guidewire 10 may include a distal section 14 that may be, as is well known in the art, generally configured for probing within the anatomy of a patient. Guidewire 10 may be used for intravascular procedures according to common practice and procedure. For example, guidewire 10 may be used in conjunction with another medical device such as a catheter 16. Of course, numerous other uses are known amongst clinicians for guidewires and other similarly configured medical devices.

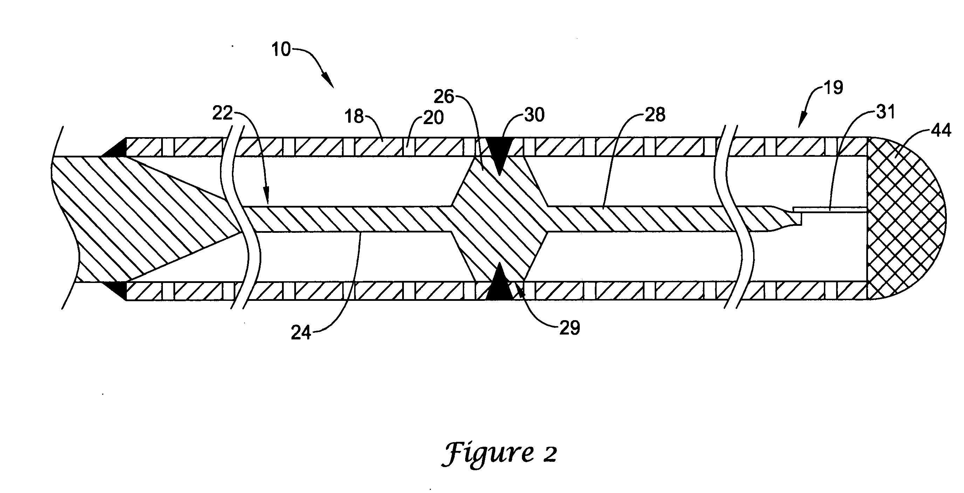

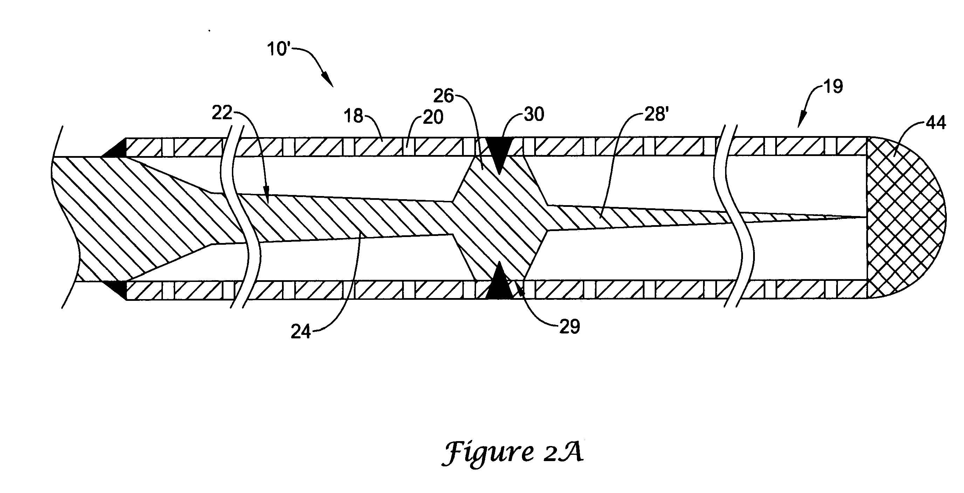

Turning now to Figure 2, here it can be seen that guidewire 10 may include a tubular member 18 having a plurality of slots 20 formed therein. A core wire 22 may be disposed within tubular member 18. A distal tip region 19 may be defined adjacent the distal end of guidewire 10. The positioning and relationship between core wire 22 and tubular member 18 may impact the overall performance characteristics of guidewire 10. Accordingly, it may be desirable for there to be one or more convenient attachment points between core wire 22 and tubular member 18. This may help to secure core wire 22 to tubular member 18. In addition, this may allow, for example, torque to be transferred from the proximal end to the distal end of guidewire 10 and between core wire 22 and tubular member 18.

The design of guidewire 10 as well as other guidewires disclosed herein incorporates structural modifications create convenient attachment points, for example, between core wire 22 and tubular member 18. For example, Figure 2 illustrates that core wire 22 has a first outer diameter region 24 and a second outer diameter region 26. First outer diameter region 24 has an outside diameter (e.g., a first outside diameter) that is smaller than the inside diameter of tubular member 18. Second outer diameter region 26, in contrast, has a second outside diameter that is substantially equal to the inside diameter of tubular member 18. In some embodiments, core wire 22 may include a third outer diameter region 28. Third outer diameter region 28 may have a third outside diameter that may be, for example, smaller than the inside diameter of tubular member 18.

In at least some embodiments, first outer diameter region 24 and third outer diameter region 28 are positioned on opposing sides of second outer diameter region 26. Accordingly, at least a region of first outer diameter region 24 and at least a region of third outer diameter region 28 are disposed within tubular member 18. Consequently, the entire length of second outer diameter region 26 is also disposed within tubular member 18. Moreover, the length of regions 24/26/28 may vary. In at least some embodiments, second outer diameter region 26 is generally shorter than either of or both of regions 24/28.

Because second outer diameter region 26 has an outside diameter that is essentially the same as the inner diameter of tubular member 18, a "point of contact" (denoted by reference number 29 in Figure 2) is defined at the intersection of core wire 22 and tubular member 18. The point of contact 29 between core wire 22 and tubular member 18 may be desirable for a number of reasons. For example, point of contact 29 may be a position where core wire 22 and tubular member 18 are attached. In addition, point of contact 29 may allow for torque and/or other forces to efficiently transfer between core wire 22 and tubular member 18.

In some embodiments, a shaping member 31 may be coupled to third outer diameter region 28 and extend distally therefrom to a tip member 44. Shaping member 31 may include, for example, a shapeable or otherwise elastic material that allows tip 19 to be bent into a desired shape. Any suitable material, however, may be utilized. Tip member 44 may comprise, for example, a solder ball or bead. In other embodiments, third outer diameter region 28 may taper. These embodiments may or may not including a shaping member 31. For example, Figure 2A illustrates guidewire 10' where third outer diameter region 28' has a continuous taper and lacks a shaping member (although, a shaping member may be utilized).

With the above discussion in mind, the methods for manufacturing guidewire 10 may include providing tubular member 18, providing core wire 22, and securing core wire 22 to tubular member 18. The securing step may include forming a frictional engagement or fit, laser welding, spot welding, mechanical bond, etc. The types of bonds contemplated are discussed in more detail below. Alternative embodiments of the securing step may include adding and/or utilizing another substance (such as the joining substance discussed below) to secure tubular member 18 and core wire 22.

In some embodiments, the outside diameter of region 26 is sufficiently close to the inside diameter of tubular member 18 such that a frictional engagement is created that secures the integrity of the bond between these structures at the point of contact 29. The frictional bond helps keep core wire 22 in contact with tubular member 18 so that torque can be efficiently transferred therebetween. In these as well as other embodiments, a laser, spot, or similar type of weld 30 may be added to secure the bond between core wire 22 and tubular member 18 and, therefore, increase the probability that torque and/or other forces can be efficiently transferred between core wire 22 and tubular member 18.

Core wire 22 may be made from any suitable material such as a metal, metal alloy, polymer, metal-polymer composite, and the like. Some examples of suitable metals and metal alloys include stainless steel, such as 304V, 304L, and 316LV stainless steel; mild steel; nickel-titanium alloy such as linear-elastic and/or superelastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; combinations thereof; and the like; or any other suitable material.

As alluded to above, within the family of commercially available nickel-titanium or nitinol alloys, is a category designated "linear elastic" which, although is similar in chemistry to conventional shape memory and superelastic varieties, exhibits distinct and useful mechanical properties. By applications of cold work, directional stress, and heat treatment, the material is fabricated in such a way that it does not display a substantial "superelastic plateau" or "flag region" in its stress/strain curve. Instead, as recoverable strain increases, the stress continues to increase in a substantially linear relationship until plastic deformation begins. In some embodiments, the linear elastic nickel-titanium alloy is an alloy that does not show any martensite/austenite phase changes that are detectable by DSC and DMTA analysis over a large temperature range.

For example, in some embodiments, there are no martensite/austenite phase changes detectable by DSC and DMTA analysis in the range of about -60°C to about 120°C. The mechanical bending properties of such material are therefore generally inert to the effect of temperature over this very broad range of temperature. In some embodiments, the mechanical properties of the alloy at ambient or room temperature are substantially the same as the mechanical properties at body temperature. In other words, across a broad temperature range, the material maintains its linear elastic characteristics and/or properties and has essentially no yield point. In some embodiments, the use of the linear elastic nickel-titanium alloy allows the medical device to exhibit superior "pushability" around tortuous anatomy. Accordingly, components of guidewire 10 such as core wire 22 or any other structure of guidewire 10 may include linear elastic nickel-titanium alloy.

In some embodiments, the linear elastic nickel-titanium alloy is in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803. In some other embodiments, a superelastic alloy, for example a superelastic nitinol can be used to achieve desired properties.

In at least some embodiments, portions or all of core wire 22 may also be doped with, made of, or otherwise include a include a radiopaque material. Radiopaque materials are understood to be materials capable of producing a relatively bright image on a fluoroscopy screen or another imaging technique during a medical procedure. This relatively bright image aids the user of guidewire 10 in determining its location. Some examples of radiopaque materials can include, but are not limited to, gold, platinum, molybdenum, palladium, tantalum, tungsten or tungsten alloy, plastic material loaded with a radiopaque filler, and the like.

In some embodiments, a degree of MRI compatibility is imparted into guidewire 10. For example, to enhance compatibility with Magnetic Resonance Imaging (MRI) machines, it may be desirable to make core wire 22 or other portions of the guidewire 10, in a manner that would impart a degree of MRI compatibility. For example, core wire 22 or portions thereof, may be made of a material that does not substantially distort the image and create substantial artifacts (artifacts are gaps in the image). Certain ferromagnetic materials, for example, may not be suitable because they may create artifacts in an MRI image. Core wire 22 or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane, polypropylene (PP), polyvinylchloride (PVC), polyetherester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID®) available from EMS American Grilon), perfluoro(propyl vinyl ether) (PFA), ethylene vinyl alcohol, polyolefin, polystyrene, epoxy, polyvinylidene chloride (PVdC), polycarbonates, ionomers, biocompatible polymers, other suitable materials, or mixtures, combinations, copolymers thereof, polymer/metal composites, and the like.

Tubular member 18 may similarly be made of a generally metallic material such as those listed above. In at least some embodiments, tubular member 18 is made from a nickel-titanium alloy (e.g., super plastic and/or shape memory nitinol). Any other suitable material may be utilized including those listed herein.

The tubular member 18 includes a plurality of cuts, apertures, and/or slots 20 formed therein. Slots 20 can be formed by methods such as micro-machining, saw-cutting (e.g., using a diamond grit embedded semiconductor dicing blade), laser cutting, electron discharge machining, grinding, milling, castling, molding, chemically etching or treating, or other known methods, and the like. In some such embodiments, the structure of the tubular member 18 is formed by cutting and/or removing portions of the tube to form slots 20. Some example embodiments of appropriate micromachining methods and other cutting methods, and structures for tubular members including slots and medical devices including tubular members are disclosed in U.S. Pat. Publication Nos. US 2003/0069522 and US 2004/0181174-A2; and U.S. Pat. Nos. 6,766,720; and 6,579,246. Some example embodiments of etching processes are described in U.S. Pat. No. 5,106,455. It should be noted that the methods for manufacturing guidewire 10 may include forming slots 20 in tubular member 18 using any of these or other manufacturing steps.

Various embodiments of arrangements and configurations of slots 20 are contemplated. In some embodiments, at least some, if not all of slots 20 are disposed at the same or a similar angle with respect to the longitudinal axis of the tubular member 18. As shown, slots 20 can be disposed at an angle that is perpendicular, or substantially perpendicular, and/or can be characterized as being disposed in a plane that is normal to the longitudinal axis of tubular member 18. However, in other embodiments, slots 20 can be disposed at an angle that is not perpendicular, and/or can be characterized as being disposed in a plane that is not normal to the longitudinal axis of tubular member 18. Additionally, a group of one or more slots 20 may be disposed at different angles relative to another group of one or more slots 20. The distribution and/or configuration of slots 20 can also include, to the extent applicable, any of those disclosed in U.S. Pat. Publication No. US 2004/0181174.

Slots 20 may be provided to enhance the flexibility of tubular member 18 while still allowing for suitable torque transmission characteristics. Slots 20 may be formed such that one or more rings and/or turns interconnected by one or more segments and/or beams are formed in tubular member 18, and such rings and beams may include portions of tubular member 18 that remain after slots 20 are formed in the body of tubular member 18. Such an interconnected ring structure may act to maintain a relatively high degree of tortional stiffness, while maintaining a desired level of lateral flexibility. In some embodiments, some adjacent slots 20 can be formed such that they include portions that overlap with each other about the circumference of tubular member 18. In other embodiments, some adjacent slots 20 can be disposed such that they do not necessarily overlap with each other, but are disposed in a pattern that provides the desired degree of lateral flexibility.

Additionally, slots 20 can be arranged along the length of, or about the circumference of, tubular member 18 to achieve desired properties. For example, adjacent slots 20, or groups of slots 20, can be arranged in a symmetrical pattern, such as being disposed essentially equally on opposite sides about the circumference of tubular member 18, or can be rotated by an angle relative to each other about the axis of tubular member 18. Additionally, adjacent slots 20, or groups of slots 20, may be equally spaced along the length of tubular member 18, or can be arranged in an increasing or decreasing density pattern, or can be arranged in a non-symmetric or irregular pattern. Other characteristics, such as slot size, slot shape and/or slot angle with respect to the longitudinal axis of tubular member 18, can also be varied along the length of tubular member 18 in order to vary the flexibility or other properties. In other embodiments, moreover, it is contemplated that the portions of the tubular member, such as a proximal section, or a distal section, or the entire tubular member 18, may not include any such slots 20.

As suggested above, slots 20 may be formed in groups of two, three, four, five, or more slots 20, which may be located at substantially the same location along the axis of tubular member 18. Within the groups of slots 20, there may be included slots 20 that are equal in size (i.e., span the same circumferential distance around tubular member 18). In some of these as well as other embodiments, at least some slots 20 in a group are unequal in size (i.e., span a different circumferential distance around tubular member 18). Longitudinally adjacent groups of slots 20 may have the same or different configurations. For example, some embodiments of tubular member 18 include slots 20 that are equal in size in a first group and then unequally sized in an adjacent group. It can be appreciated that in groups that have two slots 20 that are equal in size, the beams (i.e., the portion of tubular member 18 remaining after slots 20 are formed therein) are aligned with the center of tubular member 18. Conversely, in groups that have two slots 20 that are unequal in size, the beams are offset from the center of tubular member 18. Some embodiments of tubular member 18 include only slots 20 that are aligned with the center of tubular member 18, only slots 20 that are offset from the center of tubular member 18, or slots 20 that are aligned with the center of tubular member 18 in a first group and offset from the center of tubular member 18 in another group. The amount of offset may vary depending on the depth (or length) of slots 20 and can include essentially any suitable distance.

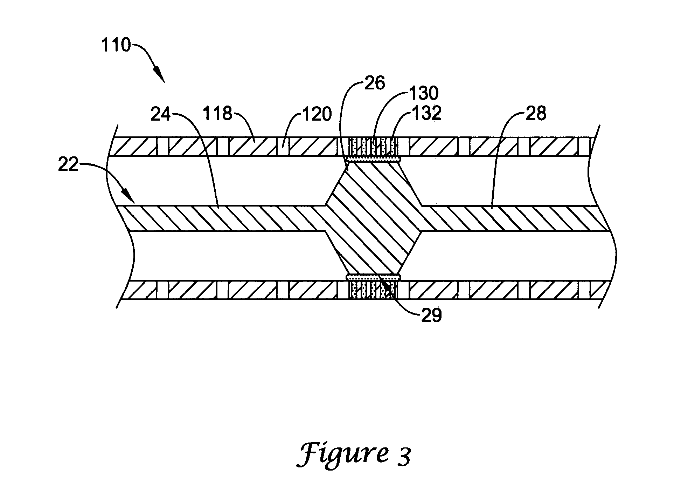

While weld 30, as shown in Figure 2, may be suitable for securing core wire 22 to tubular member 18 in a number of different embodiments, a number of alternative methods and/or substances are also contemplated for joining core wire 22 with tubular member 18. For example, a joining substance 130 such as solder, an adhesive, brazing, or the like may be utilized to secure core wire 22 to tubular member 118 in guidewire 110 (which is similar in form and function to guidewire 10 except for the noted differences), as depicted in Figure 3. Of course any number of alternative joining substances 130 may be utilized.

Figure 3 also depicts that tubular member 118 may optionally include one or more joining substance openings 132 formed therein. Tubular member 118 is otherwise similar to tubular member 18. Openings 132 allow a manufacturer to more easily pass joining substance 130 from the outside surface of tubular member 118 to the inside surface at the point of contact 29 so that joining substance 130 can secure tubular member 118 to core wire 22. Accordingly, the methods for manufacturing guidewire 110 may include passing joining substance 130 through openings 132. In alternative embodiments, the point of contact 29 may be disposed near one of the slots 120 in tubular member 118 so that joining substance 130 can be easily be disposed adjacent core wire 22 in an analogous manner.

For a number of reasons, a number of guidewires such as guidewires 10/110 may include one or more coils. In some embodiments, the coils may be useful in forming the distal tip (e.g., a distal spring tip) of the guidewire. In these and other embodiments, the coils (i.e., one or more of the coils) may also desirably impact the overall design of the guidewire. For example, the coil or coils may be made from or otherwise include a radiopaque material, an MRI compatible and/or visible material, or any other suitable material including any of those disclosed herein that may desirably impact the design of guidewire 10/110.

When designing a guidewire that includes a coil, it may be useful to consider how the coil is secured to other components of the guidewire. This might include material considerations and bond compatibility between the coil and other guidewire components. For example, the coil may be made from a material that is not easily welded to a guidewire component such as a tubular member (e.g., tubular member 18/118) that is made from a nickel-titanium alloy.

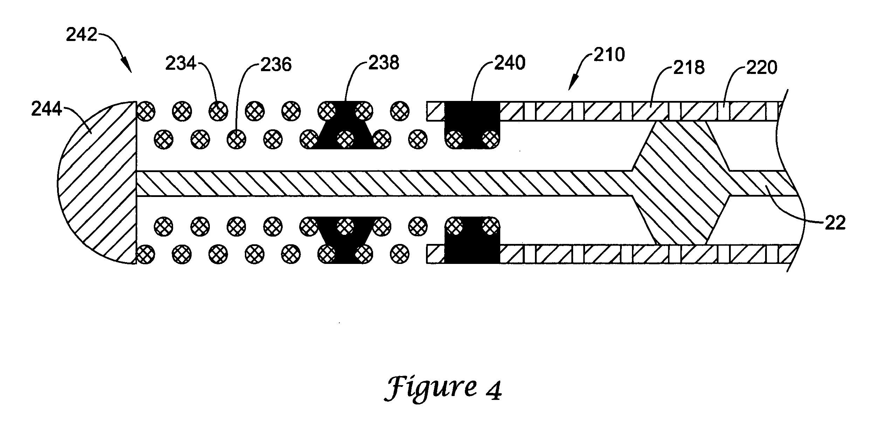

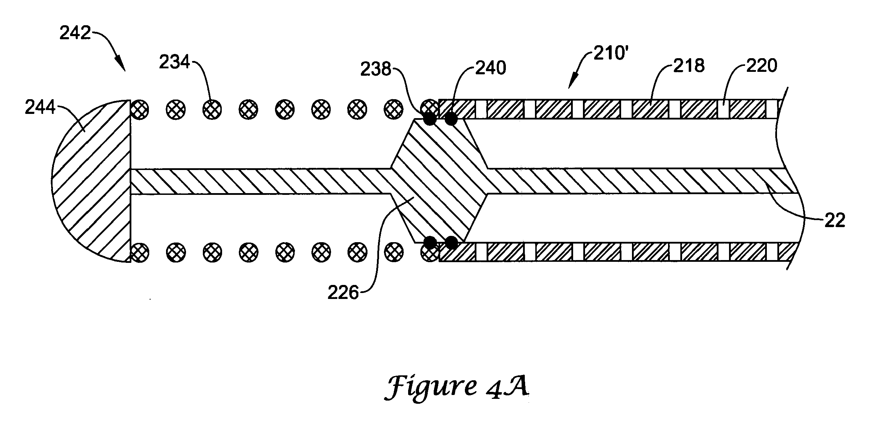

Figure 4 illustrates a guidewire 210 where a first coil 236 and a second coil 234 are secured to a tubular member 218. Guidewire 210 and tubular member 218 are similar in form and function to other similarly named structures disclosed herein. First coil 236 and second coil 234 may be secured together using a weld (e.g., laser weld, spot weld, etc.) or joining substance (adhesive, solder, etc.) bond 238. First coil 236, in turn, may be bonded with tubular member 218 using another weld (e.g., laser weld, spot weld, etc.) or joining substance (adhesive, solder, etc.) bond 240. Of course any number of alternative bonding techniques may be utilized for bonding any of the suitable structures disclosed herein including swaging, brazing, mechanically bonding, crimping, frictionally fitting or bonding, and the like, or any other suitable bonding method.

The design of guidewire 210 may be useful when one of the coils 234/236 has a lower bonding affinity for tubular member 218. For example, second coil 234 may be made from a material such as stainless steel that is not easily welded to a nickel-titanium alloy tubular member 218. First coil 236, however, may be made from a radiopaque material such as platinum, which has an increased bonding affinity for nickel-titanium alloy. Therefore, the combination of bonds 238/240 efficiently secures together first coil 236, second coil 234, and tubular member 218. The methods for manufacturing guidewire 210 may include bonding coils 234/236 with tubular member 218 in this manner.

Second coil 234 may extend distally so as to form or define a spring tip region 242 of guidewire 210, terminating with a solder ball distal tip 244. The methods for manufacturing guidewire 210 may include forming tip region 242 with coil 234 and adding distal tip 244. In some embodiments, first coil 236 may proximally terminate as shown in Figure 4 while in other embodiments coil 236 may extend further in the proximal direction. Core wire 22 may be disposed within tubular member 218 (in a manner similar to how core wire 22 is arranged in the embodiments described above) and may be coupled or attached to distal tip 244. Tubular member 218 is similar to other tubular members disclosed herein and may include a plurality of slots 220.

It can be appreciated that several coil configurations and/or arrangements are contemplated besides what is shown in Figure 4. For example, coils 234/236 may be arranged as step coils that step in outer diameter. Additionally step coils may also be added. Additionally, coils 234/236 may be formed from a single multilayer coil (e.g., where the coils are formed from a single filament that is wound in one direction and then circles back in the opposite direction) or they may multifilar coils may be utilized. Some examples of coil configurations and/or arrangements that may be utilized are disclosed in U.S. Patent No. 7,182,735.

Furthermore, other embodiments of guidewire 210 may alter the relative position of core wire 22 relative to tubular member 218. For example, Figure 4A illustrates guidewire 210' where second outer diameter region 226 is positioned adjacent the distal end of tubular member 220. Bond 240 may be disposed between and bond second outer diameter region 226 and coil 238. Bond 238, which may be disposed distal of bond 240, may bond coil 234 (and/or coil 236, which may be present in some embodiments) with second outer diameter region 226.

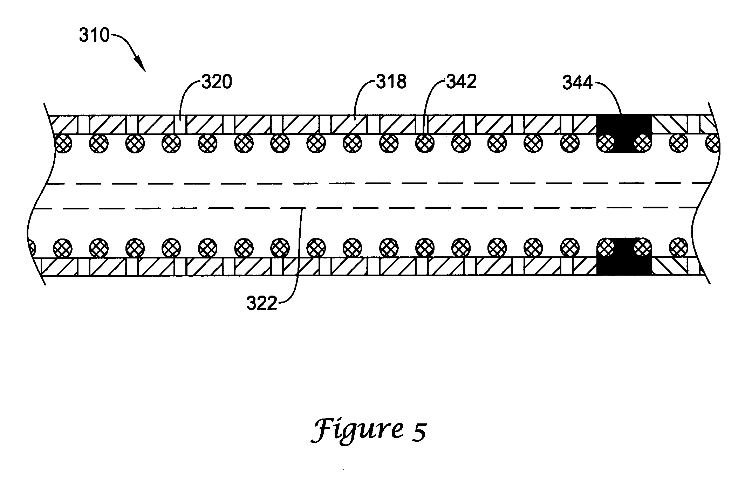

Figure 5 illustrates another example guidewire 310 that is similar to guidewire 210. Guidewire 310 includes a coil 342 that is secured along the inside surface of tubular member 318 using a weld (e.g., laser weld, spot weld, etc.) or joining substance (adhesive, solder, etc.) bond 344. In some embodiments, coil 342 is made from a radiopaque material so that the bonding of coil 342 to tubular member 318 8 adds desirable fluoroscopic properties to tubular member 318. Other embodiments utilize alternative coils that may attribute different or additional properties to tubular member 318. Tubular member 318 is similar to other tubular members disclosed herein and may include a plurality of slots 320. A core wire 322 (shown in phantom) may be disposed within tubular member 318 and may be similar to other core wires described herein.

In some embodiments, coil 342 may actually be a proximal portion of first coil 236. According to this embodiment, Figure 5 can be thought of as illustrating how coil 236 (i.e., proximal portion 342) is secured at a more proximal location within tubular member 318. Alternatively, coil 342 may simply be a coil disposed in tubular member 318 that is shown to illustrate the method for securing coil 342 to the inside surface of tubular member 318 or any other similar tubular member.

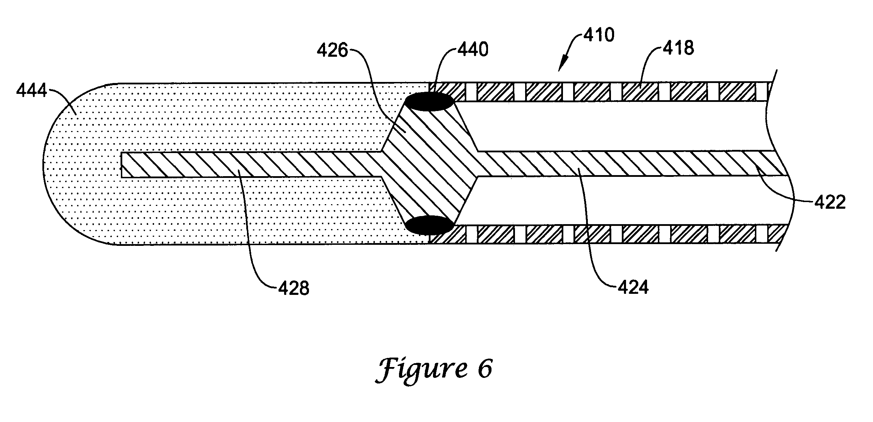

Figure 6 illustrates another example guidewire 410 that may be similar in form and function to any of the other guidewires disclosed herein. Guidewire 410 includes core wire 422 having second outside diameter region 426 flanked by first and second outside diameter regions 424/428. Bond 440 may bond second outside diameter region 426 to slotted tubular member 418. A tip member 444 may extend distally from tubular member 418. In at least some embodiments, tip member 444 is a polymer tip that be made from any of the suitable polymers disclosed herein.

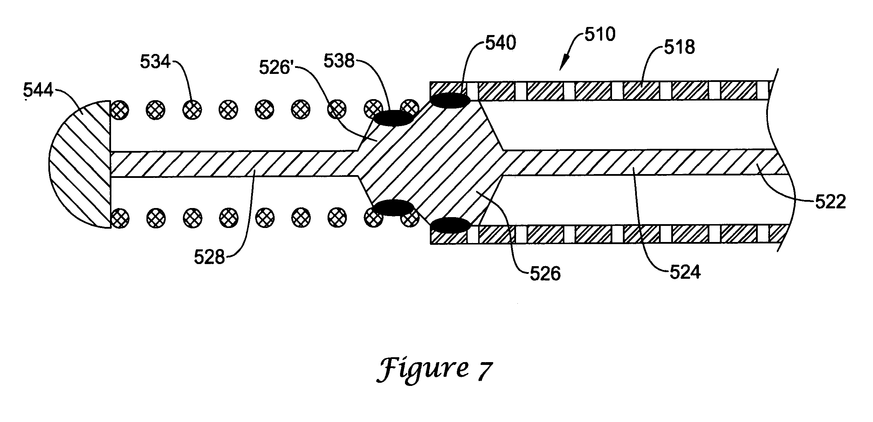

Figure 7 illustrates another example guidewire 510 that may be similar in form and function to any of the other guidewires disclosed herein. Guidewire 510 includes core wire 522 having second outside diameter region 526 flanked by first and second outside diameter regions 524/528. In addition, second outside diameter region 526 may include a shoulder region 526' where the outer diameter is reduced. Bond 540 may bond second outside diameter region 526 to slotted tubular member 520. Bond 538 may bond shoulder region 526' to coil 534. Coil 534 may form a spring tip for guidewire 510 and it may extend distally to a tip member 544 that may take the form, for example, of a solder ball tip.

It should be understood that this disclosure is, in many respects, only illustrative. Changes may be made in details, particularly in matters of shape, size, and arrangement of steps without exceeding the scope of the invention. The invention's scope is, of course, defined in the language in which the appended claims are expressed.

## Claims

1. A medical device, comprising:
a slotted tubular member (18) having a lumen expending tberethrough, an inner surface, and an inner diameter;
a core member (22) disposed in the lumen, the core member having a first outer diameter region (24), a second outer diameter region (26) and a third outer diameter region (28); and
wherein the second outer diameter region has a second outer diameter that is subamntially tbe same as the inner diameter of the slowed tubular member so that a secure point of contact is defined at the intersection of the second outer diameter region and the inner surface of the slotted tubular member.

2. The medical device of claim 1, wherein the second outer diameter region is disposed distally of the first outer diameter region, and the third outer diameter region is disposed distally of the second outer diameter region; wherein the first outer diameter region has a first outer diameter that is smaller than the second outer diameter; and wherein the third outer diameter region has a third outer diameter that is smaller than the first outer diameter.

3. The medical device of claim 1,
wherein at leat a portion of the first outer diameter region and at least a portion of the third outer diameter region are disposed within the lumen;
the device further comprising :
a first coil coupled to the slotted tubular member and extending distally therefrom; sad
a distal tip member coupled to the first coil.

4. The medical device of either of claims 1 or 2, further comprising a first coil that is coupled to the tubular member, and wherein the first coil is coupled to-the tubular member mechanically, or with a laser weld, or spot weld, or an adhesive, or solder.

5. The medical device of either of claims 3 or 4, further comprising a second coil coupled to the fast coil

6. The medical device of any of claims 1-5, wherein the first outer diameter is smaller than the inner diameter of the slotted tubular member so that a space is defined between the second outer diameter region and the inner surface of the slotted tubular member.

7. The medical device of any of claims 1-6, wherein the third outer diameter is smaller than the inner diameter of the slotted tubular member so that a space is defined between the third outer diameter region and the inner surface of the slotted tubular member.

8. The medical device of any of claims 1-7, wherein the second outer diameter region of the core member is attached to the inner surface of the slotted tubular member mechanically, or with a laser weld, or spot weld, or solder, or adhesive.

9. The medical device of any of claims 1-8, wherein the second outer diameter region includes a shoulder region having an outer diameter that is smaller than the second outer diameter.

10. The medical device of any of claims 3-9, wherein the first coil is coupled to the slotted tubular member by sa intermediate coil that is bonded to both the slotted tubular member and to the first coils

11. The medical device of any of claims 1-10, wherein the slotted tubular member includes a nickel-lunium alloy.

12. A method for manufacturing a medical device, comprising the steps of:
providing a slotted tubular member, the slotted tubular member having a lumen extending therethrough, an inner surface, and an inner diameter;
providing a core member, the core member having a first outer diameter region, a second outer diameter region, and a third outer diameter region;
disposing the core member in the lumen of the slotted tubular member, and
wherein the second outer diameter region has a second outer diameter that is substantially die same as the inner diameter of the slotted tubular member so that a secure point of contact is defined at the intersection of the second outer diameter region and the inner surface of the slotted tubular member.

13. The method of claim 12;
wherein the first outer diameter region has a first outer diameter that is smaller than the inner diameter of the slotted tubular member so that a space is defined between the first outer diameter region and the inner surface of slotted Tubular member, the method further comprising the step of
attaching the second outer diameter region of the core member to the inner surface of the slotted tubular member.

14. The method of claim 12, wherein the core member is disposed in the lumen of the slotted tubular ember so that at least a portion of the first outer diameter region and at least a portion of the third outer diameter region are disposed in the lumen; the method further comprising the steps of
costing a coil to the slotted tubular member so that a portion of the coil extends distally from the slotted tubular member; and
coupling a distal tip member to the coil.

15. The method of claim 14; wherein the step of coupling a coil to the slotted tubular member so that a portion of the coil extends distally from the slotted tubular member includes coupling an intermediate coil to the coil and bonding the intermediate coil to the slotted tubular member.

## Patentansprüche

1. Medizinische Vorrichtung, die aufweist:
ein geschlitztes röhrenförmiges Element (18), das ein sich dort hindurch erstreckendes Lumen, eine Innenoberfläche und einen Innendurchmesser aufweist;
ein im Lumen angeordnetes Kernelement (22), wobei das Kernelement einen ersten Außendurchmesserbereich (24), einen zweiten Außendurchmesserbereich (26) und einen dritten Außendurchmesserbereich (28) aufweist; und
wobei der zweite Außendurchmesserbereich einen zweiten Außendurchmesser aufweist, der im Wesentlichen derselbe wie der Innendurchmesser des geschlitzten röhrenförmigen Elements ist, so dass an der Schnittstelle des zweiten Außendurchmesserbereichs und der Innenoberfläche des geschlitzten röhrenförmigen Elements ein sicherer Berührungspunkt definiert ist.

2. Medizinische Vorrichtung nach Anspruch 1, wobei der zweite Außendurchmesserbereich distal vom ersten Außendurchmesserbereich angeordnet ist, und der dritte Außendurchmesserbereich distal vom zweiten Außendurchmesserbereich angeordnet ist;
wobei der erste Außendurchmesserbereich einen ersten Außendurchmesser aufweist, der kleiner als der zweite Außendurchmesser ist; und
wobei der dritte Außendurchmesserbereich einen dritten Außendurchmesser aufweist, der kleiner als der erste Außendurchmesser ist.

3. Medizinische Vorrichtung nach Anspruch 1, wobei mindestens ein Abschnitt des ersten Außendurchmesserbereichs und mindestens ein Abschnitt des dritten Außendurchmesserbereichs im Lumen angeordnet sind;
wobei die Vorrichtung ferner aufweist:
eine erste Spirale, die mit dem geschlitzten röhrenförmigen Element gekoppelt ist und sich distal davon erstreckt; und
ein distales Spitzenelement, das mit der ersten Spirale gekoppelt ist.

4. Medizinische Vorrichtung nach einem der Anspruche 1 oder 2, die ferner eine erste Spirale aufweist, die mit dem röhrenförmigen Element gekoppelt ist, und wobei die erste Spirale mechanisch, oder mit einer Laserschweißung, oder Punktschweißung oder einem Klebemittel oder Lötmittel mit dem röhrenförmigen Element gekoppelt ist.

5. Medizinische Vorrichtung nach einem der Ansprüche 3 oder 4, die ferner eine zweite Spirale aufweist, die mit der ersten Spirale gekoppelt ist.

6. Medizinische Vorrichtung nach einem der Ansprüche 1-5, wobei der erste Außendurchmesser kleiner als der Innendurchmesser des geschlitzten röhrenförmigen Elements ist, so dass zwischen dem zweiten Außendurchmesserbereich und der Innenoberfläche des geschlitzten röhrenförmigen Elements ein Raum definiert wird.

7. Medizinische Vorrichtung nach einem der Ansprüche 1-6, wobei der dritte Außendurchmesser kleiner als der Innendurchmesser des geschlitzten röhrenförmigen Elements ist, so dass zwischen dem dritten Außendurchmesserbereich und der Innenoberfläche des geschlitzten röhrenförmigen Elements ein Raum definiert wird.

8. Medizinische Vorrichtung nach einem der Ansprüche 1-7, wobei der zweite Außendurchmesserbereich des Kernelements mechanisch, oder mit einer Laserschweißung, oder Punktschweißung oder einem Klebemittel oder Lötmittel an der Innenoberfläche des geschlitzten röhrenförmigen Elements befestigt ist.

9. Medizinische Vorrichtung nach einem der Ansprüche 1-8, wobei der zweite Außendurchmesserbereich einen Schulterbereich aufweist, der einen Außendurchmesser aufweist, der kleiner als der zweite Außendurchmesser ist.

10. Medizinische Vorrichtung nach einem der Ansprüche 3-9, wobei die erste Spirale mit dem geschlitzten röhrenförmigen Element durch eine Zwischenspirale gekoppelt ist, die sowohl mit dem geschlitzten röhrenförmigen Element als auch mit der ersten Spirale verbunden ist.

11. Medizinische Vorrichtung nach einem der Ansprüche 1-10, wobei das geschlitzte röhrenförmige Element eine Nickel-Titan-Legierung aufweist.

12. Verfahren zur Herstellung einer medizinischen Vorrichtung, das die Schritte aufweist:
Bereitstellen eines geschlitzten röhrenförmigen Elements, wobei das geschlitzte röhrenförmige Element ein sich dort hindurch erstreckendes Lumen, eine Innenoberfläche und einen Innendurchmesser aufweist;
Bereitstellen eines Kernelements, wobei das Kernelement einen ersten Außendurchmesserbereich, einen zweiten Außendurchmesserbereich und einen dritten Außendurchmesserbereich aufweist;
Anordnen des Kernelements im Lumen des geschlitzten röhrenförmigen Elements; und
wobei der zweite Außendurchmesserbereich einen zweiten Außendurchmesser aufweist, der im Wesentlichen derselbe wie der Innendurchmesser des geschlitzten röhrenförmigen Elements ist, so dass an der Schnittstelle des zweiten Außendurchmesserbereichs und der Innenoberfläche des geschlitzten röhrenförmigen Elements ein sicherer Berührungspunkt definiert wird.

13. Verfahren nach Anspruch 12, wobei der erste Außendurchmesserbereich einen ersten Außendurchmesser aufweist, der kleiner als der Innendurchmesser des geschlitzten röhrenförmigen Elements ist, so dass zwischen dem ersten Außendurchmesserbereich und der Innenoberfläche des geschlitzten röhrenförmigen Elements ein Raum definiert wird; wobei das Verfahren ferner den Schritt aufweist:
Befestigen des zweiten Außendurchmesserbereichs des Kernelements an der Innenoberfläche des geschlitzten röhrenförmigen Elements.

14. Verfahren nach Anspruch 12, wobei das Kernelement im Lumen des geschlitzten röhrenförmigen Elements angeordnet wird, so dass mindestens ein Abschnitt des ersten Außendurchmesserbereichs und mindestens ein Abschnitt des dritten Außendurchmesserbereichs im Lumen angeordnet werden; wobei das Verfahren ferner die Schritte aufweist:
Koppeln einer Spirale am geschlitzten röhrenförmigen Element, so dass sich ein Abschnitt der Spirale distal vom geschlitzten röhrenförmigen Element erstreckt; und
Koppeln eines distalen Spitzenelements mit der Spirale.

15. Verfahren nach Anspruch 14, wobei der Schritt des Koppelns einer Spirale mit dem geschlitzten röhrenförmigen Element, so dass sich ein Abschnitt der Spirale distal aus dem geschlitzten röhrenförmigen Element erstreckt, das Koppeln einer Zwischenspirale an die Spirale und das Verbinden der Zwischenspirale mit dem geschlitzten röhrenförmigen Element aufweist.

## Revendications

1. Dispositif médical, comprenant :
un élément tubulaire fendu (18) avec une lumière s'étendant sur sa longueur, une surface intérieure, et un diamètre intérieur ;
un élément d'âme (22) disposé dans la lumière, ledit élément d'âme comportant une première zone de diamètre extérieur (24), une deuxième zone de diamètre extérieur (26) et une troisième zone de diamètre extérieur (28) ; et
où la deuxième zone de diamètre extérieur présente un deuxième diamètre extérieur qui est sensiblement égal au diamètre intérieur de l'élément tubulaire fendu, de manière à définir un point de contact sûr à l'intersection entre la deuxième zone de diamètre extérieur et la surface intérieure de l'élément tubulaire fendu.

2. Dispositif médical selon la revendication 1, où la deuxième zone de diamètre extérieur est disposée distalement par rapport à la première zone de diamètre extérieur, et où la troisième zone de diamètre extérieur est disposée distalement par rapport à la deuxième zone de diamètre extérieur ; où la première zone de diamètre extérieur présente un premier diamètre extérieur inférieur au deuxième diamètre extérieur ; et
où la troisième zone de diamètre extérieur présente un troisième diamètre extérieur inférieur au premier diamètre extérieur.

3. Dispositif médical selon la revendication 1, où au moins une partie de la première zone de diamètre extérieur et au moins une partie de la troisième zone de diamètre extérieur sont disposées à l'intérieur de la lumière ;
ledit dispositif comprenant en outre :
une première bobine raccordée à l'élément tubulaire fendu et s'étendant distalement depuis celui-ci ; et
un élément d'embout distal raccordé à la première bobine.

4. Dispositif médical selon la revendication 1 ou la revendication 2, comprenant en outre une première bobine raccordée à l'élément tubulaire, et où la première bobine est raccordée mécaniquement à l'élément tubulaire, ou par soudage laser, soudage par points, ou au moyen d'un adhésif ou d'un métal d'apport.

5. Dispositif médical selon la revendication 3 ou la revendication 4, comprenant en outre une deuxième bobine raccordée à la première bobine.

6. Dispositif médical selon l'une des revendications 1 à 5, où le premier diamètre extérieur est inférieur au diamètre intérieur de l'élément tubulaire fendu, de manière à définir un espace entre la deuxième zone de diamètre extérieur et la surface intérieure de l'élément tubulaire fendu.

7. Dispositif médical selon l'une des revendications 1 à 6, où le troisième diamètre extérieur est inférieur au diamètre intérieur de l'élément tubulaire fendu, de manière à définir un espace entre la troisième zone de diamètre extérieur et la surface intérieure de l'élément tubulaire fendu.

8. Dispositif médical selon l'une des revendications 1 à 7, où la deuxième zone de diamètre extérieur de l'élément d'âme est fixée mécaniquement à la surface intérieure de l'élément tubulaire fendu, ou par soudage laser, soudage par points, ou au moyen d'un métal d'apport ou d'un adhésif.

9. Dispositif médical selon l'une des revendications 1 à 8, où la deuxième zone de diamètre extérieur comporte une zone d'épaulement avec un diamètre extérieur inférieur au deuxième diamètre extérieur.

10. Dispositif médical selon l'une des revendications 3 à 9, où la première bobine est raccordée à l'élément tubulaire fendu par une bobine intercalaire qui est soudée à l'élément tubulaire fendu et à la première bobine.

11. Dispositif médical selon l'une des revendications 1 à 10, où l'élément tubulaire fendu contient un alliage nickel-titane.

12. Procédé de fabrication d'un dispositif médical, comprenant les étapes suivantes :
réalisation d'un élément tubulaire fendu, ledit élément tubulaire fendu comportant une lumière s'étendant sur sa longueur, une surface intérieure, et un diamètre intérieur ;
réalisation d'un élément d'âme, ledit élément d'âme comportant une première zone de diamètre extérieur, une deuxième zone de diamètre extérieur, et une troisième zone de diamètre extérieur ;
disposition de l'élément d'âme dans la lumière de l'élément tubulaire fendu ; et
où la deuxième zone de diamètre extérieur présente un deuxième diamètre extérieur sensiblement égal au diamètre intérieur de l'élément tubulaire fendu, de manière à définir un point of contact sûr à l'intersection entre la deuxième zone de diamètre extérieur et la surface intérieure de l'élément tubulaire fendu.

13. Procédé selon la revendication 12, où la première zone de diamètre extérieur présente un premier diamètre extérieur inférieur au diamètre intérieur de l'élément tubulaire fendu, de manière à définir un espace entre la première zone de diamètre extérieur et la surface intérieure de l'élément tubulaire fendu ; ledit procédé comprenant en outre l'étape suivante :
fixation de la deuxième zone de diamètre extérieur de l'élément d'âme contre la surface intérieure de l'élément tubulaire fendu.

14. Procédé selon la revendication 12, où l'élément d'âme est disposé dans la lumière de l'élément tubulaire fendu, de telle manière qu'au moins une partie de la première zone de diamètre extérieur et au moins une partie de la troisième zone de diamètre extérieur sont disposées dans la lumière ; ledit procédé comprenant en outre les étapes suivantes :
raccordement d'une bobine à l'élément tubulaire fendu, de telle manière qu'une partie de la bobine s'étend distalement depuis l'élément tubulaire fendu ; et
raccordement d'un élément d'embout distal à la bobine.

15. Procédé selon la revendication 14, où l'étape de raccordement d'une bobine à l'élément tubulaire fendu de telle manière qu'une partie de la bobine s'étend distalement depuis l'élément tubulaire fendu comprend le raccordement d'une bobine intercalaire à la bobine et le soudage de la bobine intercalaire sur l'élément tubulaire fendu.
